# EUROPEAN PATENT SPECIFICATION

(11) **EP 1 878 431 B1**
(45) Date of publication and mention of the grant of the patent: **06.04.2011**
(21) Application number: 06747757.0
(22) Date of filing: 19.04.2006
(51) Int. Cl.: A61K 31/708, A61K 31/7084, A61K 31/4425, A61K 9/00, A61P 9/04, A61P 9/10, A61P 9/12, A61P 43/00, A61K 45/06

(54) **MEDICINAL AGENT FOR PREVENTING PROGRESSION OF APOPTOTIC AND JUGULATING NECROTIC CHANGES IN THE ORGANISM TISSUES**
MEDIZINISCHES MITTEL ZUR VERHINDERUNG DES FORTSCHREITENS APOPTOTISCHER UND KUPIERENDER NEKROTISCHER VERÄNDERUNGEN IN DEN ORGANISMUSGEWEBEN
MEDICAMENT DESTINE A LA PREVENTION DE LA PROGRESSION DE CHANGEMENTS APOPTOTIQUES ET A L'INTERRUPTION DE CHANGEMENTS NECROTIQUES DANS LES TISSUS DU CORPS

(30) Priority: 28.04.2005 RU 2005112851
(43) Date of publication of application: 16.01.2008
(73) Proprietor: Obschestvo S Ogranichennoi Otvetstvennostyu Nauchno-Proizvodstvennaya Kompania "Farmasoft", 115280 Moscow (RU)
(72) Inventor: Sukoyan, Galina Victorovna, 119034 Moscow (RU); Kharebava, Tengiz Givievich, 119454 Moscow (RU)
(74) Representative: Henriksson, Dan Ragnar Mikael
(86) International application number: PCT/RU2006/000198
(87) International publication number: WO 2006/115436

(56) References cited:
- WO-A-2004/022039
- WO-A-2005/003150
- WO-A1-99/06042
- RU-A- 98 104 073
- RU-A- 2000 108 431
- RU-C1- 2 168 993
- RU-C1- 2 205 640
- RU-C1- 2 250 210
- US-A- 5 668 114
- GALENKO-YAROSHEVSKII V P ET AL: "Antihypoxic and Antinecrotic Effect of Mexidol in Skin Ischemia" 1 February 2005 (2005-02-01), BULLETIN OF EXPERIMENTAL BIOLOGY AND MEDICINE, KLUWER ACADEMIC PUBLISHERS-PLENUM PUBLISHERS, NE, PAGE(S) 202 - 206 , XP019217961 ISSN: 1573-8221 * abstract *

## Description

This invention relates to the medicine, particularly, to medical agents aimed at the prevention of the progression of apoptotic and jugulating necrotic changes in the tissues of organisms in different etiologically and pathogenetically non-identical pathological states of the organism.

Apoptosis, or programmed cell loss, was described for the first time by J.Kerr and his colleagues in 1972 [1]. This process is an evolutionally developed physiological mechanism that, unlike necrosis, regulates the cell mass and architecture of many tissues. Apoptotic processes firstly were defined as changes due to programmed cell loss and characterized the aging process as a result of an energy synthesis decrease in organisms. In recent years, it was supposed that the acceleration of apoptotic processes characterize such different pathological processes as tissue destruction and high-grade local or general hypoxia, chronic poisoning, ischemic and reperfusion damage, atherosclerosis, arterial hypertension, congestive and chronic cardiac failure, irrespective of etiology [2, 3], as well as MIELAS syndrome and other kinds of mitochondrial deficiency deforming osteoarthritis and atrophic arthritis, gestosis, radiation sickness, and other sicknesses concerning bad ecology and toxic lesions. Activation of apoptotic changes that in the case of coupling with hypoxic-ischemic damage inevitably turns into necrotic ones, subsequently lead to cell loss, destruction of hypoxic-ischemic tissures, central nervous system dysfunction, respiratory deficiency, diuresis decrease, adiaphoresis to the therapy used, and death. For example, it was established that cardiomyocytes loss during cardiac arrest increases 10⁹ times. Programmed cell loss participates in the postnatal morphogenesis of the cardiovascular system: sinoauricular node and atrioventricular node in the development of paroxysmal arrhythmia and a sequence [4]. Apoptosis of pacemaker cells can play a role in the genesis of sudden coronary death. Nowadays, one can be sure that a major contribution to total cell loss during atherosclerosis is made by apoptosis [5, 6]. All cellular elements found in atherosclerosis plaques are subject to programmed loss [7]. It also turned out that the apoptotic index of normal media coronary vessels is 3+1%, in intima - 8+1%. In atheroma, he apoptotic index media didn't change statistically (5+1%) while in intima it increased nearly fourfold (34+5%) [8]. Thus, apoptosis myocytes and other cell inhibition can be a key mechanism for disease prevention and reduction, which is a new therapeutic target in the multi-componential therapy of the stated diseases, which enables a significant increase in the probability of survival and the quality of cells, tissues, and the organism's life.

Apoptotic processes during coronary atherosclerosis formation are mostly investigated. Inhibitors of different points in cascades of apoptotic reactions claim to be medications (table 1):

**Table 1**

| **List of the main apoptosis inhibitors** | |
|---|---|
| Physiological factor inhibitors of different apoptosis cascades | Medication |
| 1. Growth factor | 1. Calpain inhibitors |
| 2. Extra cellular matrix | 2. New proteases cyst inhibitors |
| 3. Neutral amino acids | 3. Caspase inhibitors |
| 4. Estrogens | 4. Carcinogenic promoters (PMA) |
| 5. Androgens | 5. Phenobarbital |
| 6.IL-9 | 6. Nicotine |
| 7. Antiphlogistic cytokines | |
| 8. Bcl-2 | |

Role of caspases in programmed cell loss is now being studied intensively under conditions of the development of coronary failure. H.Yaoita and colleagues demonstrated that Z-VAD-fmk, a general caspase inhibitor, is able to inhibit the processes of cardiomyocytes apoptosis and the area of myocardial infarction in rats that are under reperfusion *in vivo* [8]. A number of research studies demonstrate that caspases participate in the process of cytochrome C release during hypoxia and cardiomyocytes apoptosis induction [9, 10]. Recent research shows an increase of caspases and the TNFa level in patients with coronary failure cardiomyocytes, including cases with cardiomyopathy.

Today, there are pharmacological agents that are able to inhibit cardiomyocytes apoptosis efficiently with a different stimulus: ischemia/reperfusion, H₂O₂, TNFa etc. However, these agents (ZVAD-fmk, SB 203580, PD 98059, insulin-like growth factor, N-acetyl-cystein) are mainly used under experimental conditions. Regarding this, some prospects are connected with the further clinical research of carvedylol (1-[9H-carbazol-4-yloxy]-3-[-(methoxyphenoxy)ethyl-2-propanol). This agent is a new generation β-blocker with high-grade antioxidant action with mild vasorelaxant activity. Clinical research shows a significant decrease of those patients with a coronary failure death-rate due to carvedylol. Suppression of the Fas-receptor expression on cardiomyocytes is the mechanism of the anti-apoptotic action of the agent [12]. Some prospects for a further correction of apoptosis treatment during cardiopathology can be connected with low-molecular caspase inhibitors.

In conclusion, specific anti-apoptotic in turn means that it must "work" on nearly all process levels because there is a single mechanism controlling the apoptosis of all cells inside tissues or organisms as a whole. Moreover, this mechanism fails during different pathological processes.

But none of the abovementioned agents consider the simultaneous balanced action on different units of such a complex universal mechanism, and as a result it fails to slow down apoptosis, and is especially important for reducing its transition to tissue necrotization.

**The present invention's task** is to create a highly effective means for the prevention of apoptotic changes to the progress and jugulating necrotic changes in the organism's tissues during the pathological processes of different etiology, which is possesses low toxicity and has a wide spectrum of therapeutic uses, and which allows the removal (or minimization) of the causes of apoptotic change activation, and the decreased depth of necrotic tissue damage during pathological processes of different etiology.

The present invention relates to a composition which comprises
at least one 3-oxypyridine or derivative of 3-oxypyridine, which derivative is chosen from the group comprising 3-oxy-2-methoxypyridine, 3-oxy-2-phenylpyridine, 3-oxy-6-methyl-2-ethylpyridine and pharmaceutically acceptable salts thereof,
at least one purine or derivative of purine, chosen from the group comprising inosine, adenosine and hypoxanthine, and
nicotinamide-adenine dinucleotide,
for use in treatment of hypoxia, chronic poisoning, ischemic and reperfusion damage, atherosclerosis, arterial hypertension, congestive and chronic cardiac failure.

The present invention also relates to the use of a composition comprising
at least one 3-oxypyridine or derivative of 3-oxypyridine, which derivative is chosen from the group comprising 3-oxy-2-methoxypyridine, 3-oxy-2-phenylpyridine, 3-oxy-6-methyl-2-ethylpyridine and pharmaceutically acceptable salts thereof,
at least one purine or derivative of purine, chosen from the group consisting of inosine, adenosine, and hypoxanthine, and
nicotinamide-adenine dinucleotide,
for the manufacture of a medicament for treatment of hypoxia, chronic poisoning, ischemic and reperfusion damage, atherosclerosis, arterial hypertension, congestive and chronic cardiac failure.

The medication disclosed in the following text is in some aspects to be regarded as a composition.

**The main point of the invention** is to create medication that consists of at least one 3-oxypyridine or its derivative, at least one purine or its derivative, along with amidadenindinucleotide.

The medication is characterized by the fact that a 3-oxypyridine derivative is selected from the group including 3-oxy-2-metoxypyridine, 3-oxy-2-phenylpyridine, 3-oxy-6-methyl-2-ethylpyridine, and its pharmaceutically acceptable salts.

This medication is characterized by the fact that the 3-oxypyridine derivative is selected from the group including 3-oxy-6-methyl-2-ethylpyridine succinate, 3-oxy-6-methyl-2-ethylpyridine furamate and 3-oxy-6-methyl-2-ethylpyridine hydrochloride.

The medication is characterized by the fact that the 3-oxypyridine derivative is 3-oxy-6-methyl-ethylpyridine hydrochloride or 3-oxy-6-methyl-2-ethylpyridine succinate.

The medication is characterized by the fact that the derivative of 3-oxypyridine is 3-oxy-6-methyl-2-ethylpyridine succinate.

The medication is characterized by the fact that it contains at least one purine or its derivative selected from the group including inosine, adenosine, and hypoxanthine.

The medication is characterized by the fact that it contains inosine as a purine derivative. The medication is characterized by the fact that it contains the stated components with the following ratio: 3-oxypyridine or its derivative, from approximately 25 to approximately 1,500, nicotine-amide-adenine-dinucleotide, from approximately 0.5 to approximately 100, purine or its derivative, from approximately 100 to approximately 1,200.

The medication is characterized by the fact that, in addition, it contains L-carnitine or its derivative from approximately 10 mg to approximately 100 mg.

The medication is characterized by the fact that it contains L-acetylcarnitine as an L-carnitine derivative.

The medication is characterized by the fact that it also contains choline alphosceratus from approximately 50 mg to approximately 1,000 mg.

The medication is characterized by the fact that it also contains a pharmaceutically acceptable carrier.

The medication is characterized by the fact that it is made in the form of an injection medication.

The medication is characterized by the fact that it is in the form of lyophilizate.

The medication is characterized by the fact that it is made in the form of a solid medication.

The medication is characterized by the fact that it is made in the form of pills or pills a coating.

The medication is characterized by the fact that it is made in the form of a solid capsule. The medication is characterized by the fact that it is made in the form of a suppository. Using the invention enables the achievement of the following **technical result**:
The designed medication is a highly effective low-toxic agent that can reduce the transition of apoptotic processes into necrotic and/or inhibit apoptosis acceleration, significantly increase the organism's ability to perform physical activity, a lifetime in high altitudes and at low depths, decrease in the area of myocardial or cerebral infarction/ischemia, post-reperfusional consequential damage, endogenous and exogenous intoxication, reduction of disease duration and an increase in the probability of the survival of patients with cardiac insufficiency, along with a significant decrease in the risks of cardiosurgical and surgical operations, creation of the conditions for tissue storage during transplantation and preparation for stem cell transplantation.
The technical effect of the designed original combination is achieved due to the elimination and/or reduction of apoptosis intensity and its transition into necrotic cells, tissues, or organ damage as a result of the synergy of the acting components, which is shown in examples 1-3.

For the first time the effect of apoptosis deceleration and the reduction of its transition into necrotic combinations of ingredients for everyone has been achieved, of which, except for 3-oxypyridine derivatives, there was anti-apoptotic action shown but of which none of them have been used in medical practice thus far for this purpose.

3-oxypyridine derivatives, and particularly 3-hydroxy-6-methyl-2-ethylpyridine succinate, have anti-apoptotic properties, and apparently form a "trap" for free radicals, in which active forms of oxygen play an important role in the apoptotic processes' progress. 3-oxypyridine derivatives influence the regulation of functional metabolic cellular activity due to the inhibition of free-radical membranes' oxidation, their influence on the content of biogenic amines and the energy exchange of cells. 3-hydroxy-6-methyl-2-ethylpyridine succinate stabilizes biological membranes, increases the content of the polar fractions of glycerophospholipids, decreases the cholesterol/phospholipids ratio and thereby decreases the viscosity of the lipidic layer. The ability of 3-oxypyridine derivatives to inhibit phosphodiesterase activity and increase the content of cyclic nucleotides and the ability to inhibit prostaglandins and leukotrienes plays an important role.

The maintenance of the nicotine-amide-adenine-dinucleotide high level (NAD) that is a part of the designed medication and redox-potential of NAD/NADN is a key mechanism of apoptotic process acceleration and their transition into necrotic change inhibition. It turned out that the destructive changes in a core that was launched by apoptosis as a result of poly-ADF-ribose polymerase activation exhausted the NAD level, which is a trigger for the necrotic death of cells [11, 12]. Inosine, as its predecessor hypoxanthine, and its degradation product adenosine, are also capable of the dose-dependent inhibition of poly-ADF-ribose polymerase activation in macrophages [13]. Moreover, NAD and inosine and 3-hydroxy-6-methyl-2-ethylpyridine succinate restore the various units in a cell's energy support system, and thereby increasing oxygen use for ATP synthesis, on the one hand, and inhibiting free radicals formation, which are themselves powerful apoptosis and necrosis inductors, on the other hand.

L-carnitine strengthens apoptosis acceleration inhibition through stabilizing the mitochondrial membrane, influencing ceramide metastasis, inhibition of free radicals' formation and a positive effect on the reserve abilities of the immune system. The anti-apoptotic effect of L-carnitine and its derivatives is clearly shown on models *in vitro,* and *in vivo* [14, 15, 16, 17]. Based on these properties, the combination was strengthened during ischemic-reperfusional, hypoxic, and infectious heart damage.

The main component of choline alphosceratus, choline, is an important natural ingredient of living cells and plays a special role in human cerebral tissue. Choline is a predecessor of one of the most important human neurotransmitters, acetylcholine, and at the same time it is a donator for methyl groups and the structural component of phospholipids. A choline deficit also causes apoptosis *in vitro,* and *in vivo* [18, 19, 20], although the choline-deficit apoptosis mechanism is still unknown [21]. In considering the importance of the restoration of cholinergic regulation during destructive processes in the brain, choline alphosceratus was included in the combination during ischemic, hypoxic, and other destructive brain damage.

This designed medication demonstrates directional action on various units in the complex chain of apoptotic and necrotic reactions and as a result with its low toxicity it inhibits apoptosis acceleration, reduces the transition of apoptotic processes into necrotic ones, significantly increases organism tolerance of physical activity, lifetime at high altitudes and at low depths, decreases the area of myocardial or cerebral infarction/ischemia, post-reperfusional consequence damage, endogenous and exogenous intoxication, reduces disease duration and increases the probability of the survival of patients with cardiac insufficiency, significantly decreases the risks of cardiosurgical and surgical operations, creates the conditions for tissue storage during transplantation and the preparation for stem cell transplantation.

The numerical ratios of those included in the medication components that were used in the formula reflect the results of experimental observations that demonstrated that the overrun of the presented concentration limits decrease the synergetic action of the ingredients and thereby decrease the therapeutic efficiency of the medication.

This new agent is offered for sale in glass or plastic flacons or ampoules containing transparent fluid or white dried powder (in the case that the solvent can be included) or as enteric and sublingual pills of white color, and a suppository with the following mixture of components on 1 flacon-ampoule or pill or suppository:
**Example 1.** Ingredients are placed into a mixer in the following ratios, mg:
   3-hydroxy-6-methyl-2-ethylpyridine succinate - 25
   nicotine-amide-adenine-dinucleotide - 0.5
   inosine - 100
      They are mixed thoroughly thereafter.
**Example 2.** Ingredients are placed into a mixer in the following ratios, mg:
   3-hydroxy-6-methyl-2-ethylpyridine succinate - 1,500
   nicotine-amide-adenine-dinucleotide - 10
   inosine - 1,200
      They are mixed thoroughly thereafter.
**Example 3.** Ingredients are placed into a mixer in the following ratios, mg:
   3-hydroxy-6-methyl-2-ethylpyridine succinate - 25
   nicotine-amide-adenine-dinucleotide - 0.5
   inosine - 100
   L-carnitine - 10
      They are mixed thoroughly thereafter.
**Example 4.** Ingredients are placed into a mixer in the following ratios, mg:
   3-hydroxy-6-methyl-2-ethylpyridine succinate - 1,500
   nicotine-amide-adenine-dinucleotide - 10
   inosine -1,200
   L-carnitine - 100
      They are mixed thoroughly thereafter.
**Example 5.** Ingredients are placed into a mixer in the following ratios, mg:
   3-hydroxy-6-methyl-2-ethylpyridine succinate - 25
   nicotine-amide-adenine-dinucleotide - 0.5
   inosine - 100
   choline alphosceratus - 50
      They are mixed thoroughly thereafter.
**Example 6.** Ingredients are laced into a mixer in the following ratios, mg:
   3-hydroxy-6-methyl-2-ethylpyridine succinate -1,500
   nicotine-amide-adenine-dinucleotide -10
   inosine -1,200
   choline alphosceratus -1,000
      They are mixed thoroughly thereafter.

An agent for preventing apoptotic progress and jugulating necrotic changes in tissues is prepared in accordance with the type of pharmaceutical form as described hereafter:
To prepare an injection form, the ingredients are mixed in the stipulated proportions, the mixture is diluted in clear water that is commonly used in the pharmaceutical industry, auxiliary substances are then added. Then, the mixture is passed through Millipore filters with a 22 mkm diameter and then it's poured into ampoules or flacons that are checked for impermeability and sterility. An option of a dried injection form is possible (more stable), wherein in this case the agent in ampoules or flacons is dried within special freeze driers.
To prepare a solid dose pharmaceutical form of the agent, particularly as enteric pills, one should form the mixture into pills and coat them in gastric resistant capsules (enteric pills) or form granules and particles that are then covered with gastric resistant capsules or form medications in a mixture with gastric resistant material (durules). While making pills that are to be used in the oral cavity one should make uncoated pills produced according to special technology in order to release the pharmaceutical substance(s) in the oral cavity to provide local or general resorptive action (cheek pills, sublingual pills, etc.). Pill production with a modified release is possible - coated or uncoated pills containing special auxiliary substances or produced according to a special technology that allows the programming of the speed or area of release of the pharmaceutical substance.

Auxiliary substances are used during the production that are permitted by USSR Pharmacopeia, XI edition. The preliminary ingredients' granulation (moist granulation) technologies are preferable.

Bases, which are provided by USSR Pharmacopeia, XI edition, are used while making pharmaceutical substances in the form of a suppository (solid dose pharmaceutical form consisting of base and pharmaceutical substances that melt (dissolves, dissociates) at body temperatures).

The manufacturing method is realized in aseptic conditions in accordance with an officinal medicine control study. The presented substance, independent from its release form, is highly stable.

The pharmaceutical substance is used by a patient with different nosologies, the pathogenetic mechanisms of which include a hypoxic component and/or aptosis progress and/or necrotic changes depending on the severity of the illness. Use as adaptogenic therapy is possible for the prophylaxis of organic changes' development in different tissues of athletes, pilots, submariners, personnel working with sources of microwave frequencies, radio and telecommunication systems, children who are often ill, and teenagers. One dose of the medication contains an active principle ingredient weight ratio, which doesn't exceed the limits of the following:
3-hydroxy-6-methyl-2-ethylpyridine succinate - 25-1,500
nicotine-amide-adenine-dinucleotide - 0.5-100
inosine -100-1,200

The dose varies from once to 4 times a day (every 6 hours) depending on desiase severity.

During ischemic-reperfusional, hypoxic, and infectious heart damage, a complicated and non-complicated coronary insufficiency pharmaceutical substance is used in the form of injections, pills, or suppositories, the active principle of which contains L-carnitine or its derivatives with the following components' proportions, mg:
L-carnitine or its derivatives - 10-100
2-ethyl-3-methyl-oxypyridine succinate - 25-1,500
nicotine-amide-adenine-dinucleotide - 0.5-100
inosine - 100-1,200

The dose varies from one to 4 times a day (every 6 hours) depending on desiase severity.

During ischemic, hypoxic, and other degenerative brain damage, the pharmaceutical substance is used in the form of injections, pills, or suppositories with the active principle of which contains, mg:
Choline alphosceratus - 50-1,000
3-hydroxy-6-methyl-2-ethylpyridine succinat - 25-1,500
nicotine-amide-adenine-dinucleotide - 0.5-100
inosine - 100-1,200

Dose varies from one to 4 times a day (every 6 hours) depending on desiase severity.

The apoptosis activity was judged by poly-(ADP-ribose)-polymerase (key ferment of apoptotic cell loss), which was determined using Amersham Pharmacia Biotech test sets. The extent of necrotic cardiomyocites damage was estimated according to the quantity of myoglobin entry in the blood, in which the myoglobin content was measured spectrophotometrically.

**Example 7.** Acute hypobaric anoxia (as one of the most adequate methods of hypoxia study) in combination with hypercapnia was modeled by "lifting" low-resistant to hypoxia animals to a critical altitude of 11,500 m at a speed of 1,200 m/min. If at this altitude for more than 10 minutes, hypoxia modeling stopped, and the animals were considered conditionally "high-resistant", and animals that lived less than 10 minutes were considered conditionally "low-resistant". In the present study, only animals with low-resistance to hypoxia were used. During the second experiment of "lifting" animals in order to simulate hypobaric anoxia, in combination with hypercapnia, these parameters were recorded: loss of pose time (LPT), lifetime - time from the end of "lifting" until respiratory standstill or the first atonal inhalation occurrence, restitution time (RT) - from a respiratory standstill until active pose restoration after descending the animal. The hypoxia stability factor was calculated as a ratio of the duration to the restitution time, an increase of which means the growth of the organism's resistance to the occurrence of extreme oxygen deprivation.

The studied substances were injected intravenously (method that reflects the maximum effect of the medication) 15 minutes prior to the experiment. The designed medication was also injected intravenously in the form of an infusion or pills that were administered orally or a suppository.

Normally in myocardium poly-ADP-ribose activity in myocardium there is 0,035±0,006 pkMole/mg of protein, and in the brain - 0,030+0,004 pkMole/mg of protein.

As the study showed, the effect of the designed medication is not just the sum of all the ingredients. There was expressed a positive anti-apoptotic synergy (the sum of the separate ingredients' effects in the same doses is nearly 2 times lower than in the designed medication). The effect doesn't change significantly when the optimal ingredients ratio is used.

**Table 1**

| **Apoptosis activation and level of host defences at a sharp hypobaric hypoxia coupled with hypercapnia** | | | | | | | |
|---|---|---|---|---|---|---|---|
| Index/ medication | Dose, mg/kg | Duration, sec | Loss of Pose Time, sec | Restitution time, sec | Stability factor | PARP activity, % of control | |
| | | | | | | myocardium | brain |
| inspection | | 507±45 | 56±9 | 542±69 | 0.9±0.2 | 245±26 | 356±42 |
| 3-oxy-3-methyl-6-oxypyridine succinate | 50 | 778±107* | 136±22* | 680±69 | 1.14±0.12 | 204±21* | 312±29 |
| | 100 | 707±145 | 156±29* | 642±69 | 1.10±0.13 | 208±29 | 317±21 |
| | 500 | 784±107 | 136±39* | 690±83 | 1.14±0.10 | 202±19 | 302±32 |
| hutimine | 100 | 986±87* | 186±32* | 685±82 | 1.45±0.12* | 212±23 | 290±83 |
| succinic acid | 100 | 747±90* | 121±34 | 1042±69* | 1.0±0.2 | 225±22 | 325±20 |
| inosine | 100 | 754±30* | 186±39* | 680±69 | 1.16±0.16 | 192±23* | 242±24* |
| NAD | 1.0 | 2034±234*** | 936±79* | 1042±44 | 1.95±0.25** | 100±29** | 180±23** |
| | 10.0 | 2077±109*** | 1036±91* | 1000±82 | 2.08±0.22** | 103±24** | 142±44** |
| Lithium oxybutyrate | 100 | 1345±89** | 436+90** | 885±82 | 1.4±0.2** | 200±33 | 300±82 |
| 3-hydroxy-6-methyl-2-ethylpyridine + inosine | 50+100 | 1838±1144*** | 739±71*** | 1042±44** | 1.76±0.14** | 185±82** | 289±18* |
| 3-hydroxy-6-methyl-2-ethylpyridine +NAD | 50+1.0 | 2577±112*** | 1136±79** | 868±82* | 2.96±0.12*** | 104±14** | 132±20** |
| 3-hydroxy-6-methyl-2-ethylpyridine succinate+NAD+inosine in form of intravenous infusion in 0,2 ml of physiological solution | 50+1.0+100 | 5745±89*** | 2336±109*** | 885±82 | 6.5±0.2*** | 44±4*** | 32±6*** |
| 3-hydroxy-6-methyl-2-ethylpyridine succinate+NAD+inosine in tablet form | 50+1.0+100 | 5684±71*** | 2340±91*** | 906±62 | 6.3±0.3*** | 40±7*** | 29±2*** |
| 3-hydroxy-6-methyl-2-ethylpyridine succinate+NAD+ inosine in suppository form | 50+1.0+100 | 5750±75*** | 2389±78*** | 897±63 | 6.4±0.2*** | 46±14*** | 34±2*** |

| | | | | | | | |
|---|---|---|---|---|---|---|---|
| The note: comparison of the averages' distinctions: *- with norm, ^{#}- with the control. | | | | | | | |

### Example 8. Apoptosis reduction during myocardium ischemia with arterial hypertension

Arterial hypertensia of normotensive rats was reproduced by imposing the ligature in the form of a titanic heliciform ring on a site of an aorta with the arteries of kidneys departing from it. The reached narrowing of an aortic lumen was 2/3 in diameter. Animals were operated on under anesthetic, in which their abdominal cavity was opened. The arterial pressure was measured by use of a gauge with a rubber cuff on the animal's tail. A signal from the gauge was recorded on a Mingograph EMT 118. On the 30th day after the operation, all of the animals were divided into two groups: in the first group the animal's left descending interventricular artery at its top third was ligated - the hard ischemia of a myocardium, and in the second group, an irreversible bilateral occlusion of the general carotids was reproduced with a silk thread (see example 9). The operation has been performed as standard, in which it didn't take more than 7-10 minutes, and then the rats were quickly restored after a narcosis. Both groups of animals were euthanased on the 10th day after the operation. All of the investigated connections were introduced intravenously (this method of introduction was chosen to reflect the maximum effect of the medication) within 15 minutes after coronary artery bandaging. The developed means was also introduced intravenously in the form of an infusion in 5% glucose, or in tablet form with an oral administration, or in a kind of suppository.

**Table 2**

| **Apoptosis activation and level of host defences during a severe heart attack of the myocardium developed via arterial hypertension** **(6 hours after coronary artery bandaging)** | | | | | | |
|---|---|---|---|---|---|---|
| Index/ medication | Arrhythmia duration minutes | Ischemia zone, % of myocardium mass | Necrosis zone, % from myocardium mass | Necrosis zone, % from ischemia zone | Myoglobin content after 8 hours mcg/g dry solid matter | PARP activity |
| inspection | 977±12 | 47±4 | 37±4 | 79±4 | 167±23 | 0,435± 0,045*** |
| 3-hydroxy-6-methyl-2-ethylpyridine, 100 mg/kg | 58±9* | 39±3* | 26±9* | 67±3* | 127±12 | 0,256± 0,032** |
| L-carnitine, 75 mg/kg | 68±8* | 40±3* | 28±9* | 70±5* | 132±10 | 0,406± 0,030 |
| 3-hydroxy-6-methyl-2-ethylpyridine +inosine, 100+100 mg/kg | 46±6* | 32±3* | 17±2* | 53±6* | 96±10* | 0,205+ 0,047** |
| 3-hydroxy-6-methyl-2-ethylpyridine+ NAD, 100+1,0 mg/kg | 17±2*** | 30±4*** | 13±2*** | 47±4*** | 67±8** | 0,062± 0,006*** |
| 3-hydroxy-6-methyl-2-ethylpyridine+ NAD+inosine, 100+1,0+100 mg/ kg | 16±3*** | 21±2*** | 8±1*** | 38±3*** | 49±5*** | 0,052± 0,004*** |
| 3-hydroxy-6-methyl-2-ethylpyridine succinate+L- carnitine+NAD +inosine, 100+50+1,0+ 100 mg/kg intravenous injection | 6±4*** | 16±1*** | 4,0±0,5*** | 25±2*** | 32±4*** | 0,045± 0,003*** |
| 3-hydroxy-6-methyl-2-ethylpyridine succinate+L-carnitine+NAD +inosine, 100+50+1,0+ 100 mg/kg tablet form | 10±4*** | 17,4±1,4*** | 4,9±0,7*** | 26±3*** | 36±4*** | 0,049± 0,003*** |
| 3-hydroxy-6-methyl-2-ethylpyridine succinate + L-carnitine +NAD + inosine, 100+50+1,0+ 100 mg/kg suppository form | 8±3*** | 16±2*** | 4±2*** | 24±4***' | 31±4*** | 0,043± 0,005*** |

| | | | | | | |
|---|---|---|---|---|---|---|
| The note: comparison of averages' distinctions: *- with norm, ^{#}- with the control. | | | | | | |

The protective action of the developed means, irrespective of the method of introduction, under extreme conditions, a severe deep ischemia myocardium has shown its considerable superiority, and has revealed the synergy of the included components' operation and as a result has allowed to pass from a transmural heart attack of a myocardium to the ischemic defeat of a heart muscle that is not detrimental to life. This described positive effect was not able to be reached by a medication, 3-hydroxy-6-methyl-2-ethylpyridine succinate, or by its combination with any components separately. It is noteworthy that the ratio of the necrotizing zones to a common myocardium affected mass (an ischemia zone) is sharply reduced, which correlates with a sharp decrease in the activity of poly - (ADP- ribose)-polymerase - a marker of an apoptosis, registered at that (r 0.76, p<0,001). This means that the inhibition of an apoptotic process and its transition into necrotic changes under the influence of the patented means stops the necrotic changes in myocardium and thereby sharply limits a zone of necrosis of a myocardium, and considerably reduces the degree of an arrhythmias' manifestation.

### Example 9. Apoptosis reduction during brain ischemia that develops via arterial hypertension

The pathology reproduction is described in example 2. Neurological deficiency was estimated "blindly" (without the information from the rats' distribution with the groups) through each 30 minutes within 1 days on C.P. McGrow (1977) scales of mark estimation. On a C.P. McGrow (1977) scale Stroke Index, it increased with the advent of separate signs of neurological deficiency (the neurological point changed from 0-1 (norm) to 20 (death)). The basic signs of neurological deficiency included the restriction of the mobility of an animal, ptosis (one- or bilateral), swirling, hyperactive behavior, rotary, tonic, and clonic spasms, coma with a weak painful response or its respective absence. For the static analysis of the received data on the dynamics of neurological deficiency, the dispersive factorial analysis ANOVA was used. To estimate the lethality and separate neurological manifestations the Fisher's test was applied.

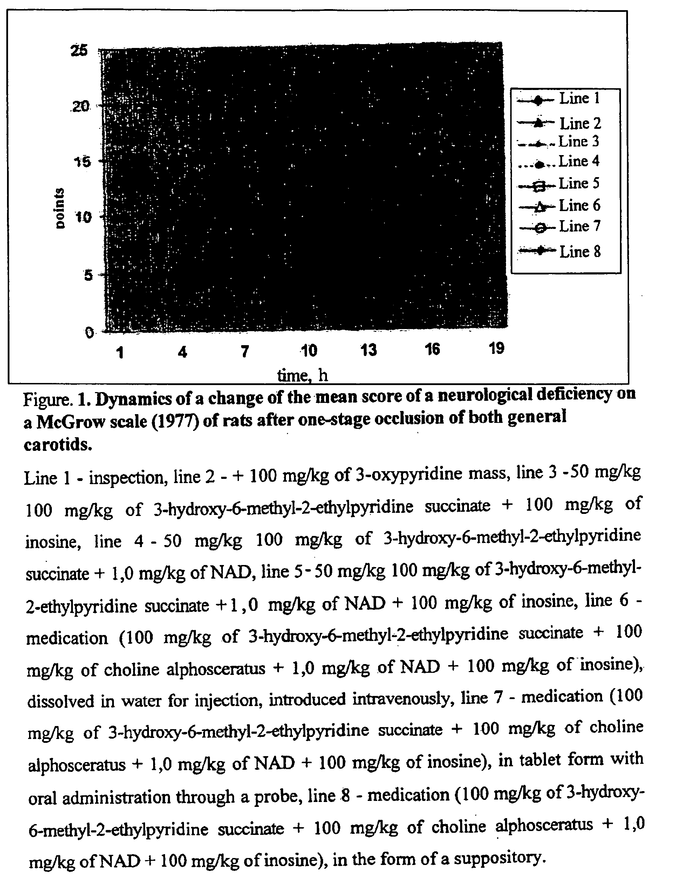

In the control group within the first 3 hours after the occlusion of the general carotids rotary and clonic spasms, 80% of the animals were registered, and in a subgroup receiving 100 mg/kg of an animal mass of 3-hydroxy-6-methyl-2-ethylpyridine succinate + 100 mg/kg of choline alphosceratus + 1,0 mg/kg of NAD + 100 mg/kg of inosine only 24% of the cases. At an estimation of under 24-hours for lethality, it is noteworthy that in the control 50% lethality was recorded at an interval up to 8 hours, whereas in the subgroups treated with the combined means 100 mg/kg of 3-hydroxy-6-methyl-2-ethylpyridine succinate + 100 mg/kg of choline alphosceratus + 1,0 mg/kg of NAD + 100 mg/kg of inosine and 100 mg/kg of 3-hydroxy-6-methyl-2-ethylpyridine succinate + 1,0 mg/kg of NAD + 100 mg/kg of inosine intervals up to 8 hours were not registered. An estimation of the lethality by the end of the first day after a global ischemia of a brain, death was the result 100% in the first subgroup, 85% in the second subgroup, 70 and 68% in the 3-1^{rd} and 4^{th} subgroups accordingly and 50 and 45% - in the 5^{th} and 6^{th} subgroups accordingly. The average life expectancy in the 1^{st} subgroup was - 8,7±1,7 h, in the 2^{nd} - 12,2±1,5 h (p<0,05), in the 3^{rd} - 14,5±2,1 h (p<0,03), in the 4^{th} - 14,2+1,9 h (p<0,01), in the 5^{th} - 18,3+1,4 h (p<0,002), and in the 6^{th} - 23,9±2,3 h (p<0,001). It is also noteworthy that by the end of the first day, a mean score on the McGrow (1977) scale for the control animals was 11 points above, versus when under the influence of the developed means 100 mg/kg of 3-hydroxy-6-methyl-2-ethylpyridine succinate + 100 mg/kg of choline alphosceratus + 1,0 mg/kg of NAD + 100 mg/kg of inosine. The obtained results enable the conclusion that the developed combination possesses the expressed ability to increase the survival rate of animals with a global ischemia of the brain and can considerably reduce the expressiveness of neurological deficiency compared to the control group (fig. 1).

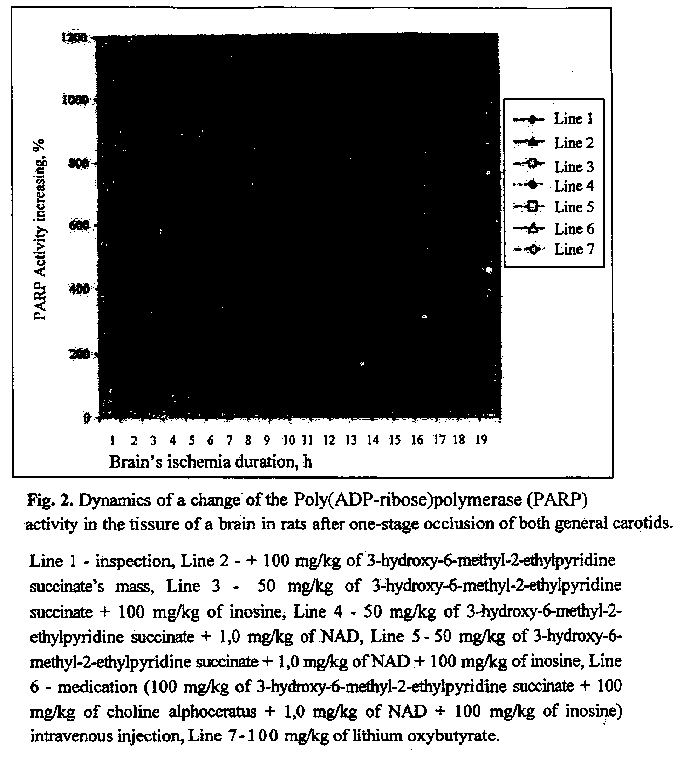

The basis of the positive effect of the created medical product irrespective of the method of introduction is its ability in essentially larger degrees to inhibit the activity of poly - (ADP-ribose)-polymerase and by that the development of apoptotic and necrotic destruction of brain cells (fig. 2).

Notice that the introduction of the developed combination reduction of neurological deficiency is accompanied by a reduction in the enzyme activity, which increases within the first day, whereas all of the preparations of comparisons render a favorable effect only within the first 6 hours after the ligation of the general carotids.

### Sources of information

1. Kerr J.F.R., Wyllie A.H., Cume A.R. Apoptosis: a basic biological phenomenon with wide-ranging implications in tissue kinetics. Br. J. Cancer 1972. 26: 239-57.
2. Colucci W.S. Apoptosis in the heart//New Engl. J. Med. 1996; 335: 1224-6. Esterbauer H., Wang G., Puhl H. Lipid peroxidation and its role in atheros-derosis. Br. Med. Bull. 1993; 49: 566-76.
3. Olivetti G., Abbi R., Quaini F. et al. Apoptosis in a failing human heart. New Engl. J. Med. 1997; 336: 1131-41.
4. James T.N. Normal and abnormal consequences of apoptosis in the human heart: from postnatal morphogenesis to paroxismal ariythrnias. Circulation 1994; 90: 556-73.
5. Han D.K.M., Haudenschild C.C., Hong U.K. et al. Evidence for Apoptosis in Human Atherogenesis and in a Rat Vascular Injury Model. Am. J. Pathol. 1995; 147: 267-77.
6. Geng Y.J., Ubby P. Evidence for Apoptosis in Advanced Human Atheroma. Colocalization with Interleukin-Ip-Converting Enzyme. Am. J. Pathol. 1995; 147: 251-66.
7. Yaoita H., Ogawa K., Maehara K., Maruyma Y. Attenuation of ischemia/reperfused injury in rats by caspase inhibitors//Circulation. 1998; 97: 276-81.
8. Haunstetter A., Izumo S. Toward antiapoptosis as a new treatment modality. Circ. Res. 2000; 86: 371-376.
9. Malhotra R., Abrosius FC III. Glucose uptake and glycolises reduce hypoxia-induced apoptosis in cultured neonatal rat cardiac myocytes. J. Biol. Chem. 1999; 274: 12567-75.
10. Yue T.L., Ohlstein E.H., Ruffolo R.R. Apoptosis: a potential target for discovering novel therapies for cardiovascular diseases. Curr. Op. Chem. Biol. 1999; 3: 474-80.
11. Yue T.L., Ma X.L., Wang X. et al. Possible involvement of stress-activates protein kinase signalling pathway and Fas receptor expression in prevention of ischemia-induced cardiomyocyte apoptosis by carvedilol. Circ. Res. 1998; 82: 166-74.
12. Chiarugi A., Moskowitz M.A. PARP-1 - a perpetrator of apoptotic cell death? Science. 2002; 297: 200-203.
13. Szabo C Dawson VL. Role of poly-(ADP-ribose)-synthetases in inflammation and ischemia-reperfusion. Trends Pharmacol. Ser. 1998; 19: 287-298.
14. Virag L., Szabo C. Purines inhibit the activation of poly-(ADP-ribose)-synthetases implications for the pathophysiology of ischemia-reperfusion injury FASEB J. 2001; 15: 99-101.
15. Albright, C.D., Lui, R., Bethea, T.C., et al. Choline deficiency induces apoptosis after hepatocyte growth factor deprivation. Biochim. Biophys. Acta. 1996; 1224: 333-341.
16. Holmes-McNary, M.Q., Loy, R., Mar, M.-H., Albright, C.D., Zeisel, S.H. Apoptosis is induced by choline deficiency in fetal brain and in PC 12 cells. Dev. Brain Res. 1997; 101: 9-16.
17. Zeisel, S.H. Choline: a nutrient that is involved in the regulation of cell proliferation, cell death, and cell transformation. Adv. Exp. Med. Biol. 1996; 399: 131-141.
18. Shin, O.H., Mar, M.H., Albright, et al. Methyl-group donors cannot prevent apoptotic death of rat hepatocytes induced by choline-deficiency. J. Cell. Biochem. 1997; 64: 196-208.
19. Andrieu-Abadie N., J.-P. Jafre'zou, Hatem S., et al. L-carnitine prevents doxorubicin-induced apoptosis of cardiac myocytes: role of inhibition of ceramide generation FASEB. 1999, 13: 1501-1510.
20. Galli, G., Fratelli, M. Activation of apoptosis by serum deprivation in a teratocarcinoma cell line: inhibition by L-acetylcarnitine. Exp. Cell Res. 1993; 204: 54-60.
21. Revoltella, R.P., Dal Canto, B., Caracciolo, L., and D'Urso, C.M. L-carnitine and some of its analogs delay the onset of apoptotic cell death initiated in murine C2.8 hepatocytic cells after hepatocyte growth factor deprivation. Biochim. Biophys. Acta. 1994; 1224: 333-341.

## Claims

1. Composition **characterized in that** it comprises
at least one 3-oxypyridine or derivative of 3-oxypyridine, which derivative is chosen from 3-oxy-2-methoxypyridine, 3-oxy-2-phenylpyridine, 3-oxy-6-methyl-2-ethylpyridine, and pharmaceutically acceptable salts thereof,
at least one purine or derivative of purine, chosen from the group comprising inosine, adenosine and hypoxanthine, and
nicotinamide-adenine dinucleotide,
for use in treatment of hypoxia, chronic poisoning, ischemic and reperfusion damage, atherosclerosis, arterial hypertension, congestive and chronic cardiac failure.

2. Composition according to claim 1, **characterized in that** the derivative of 3-oxypyridine is chosen from 3-oxy-6-methyl-2-ethylpyridine succinate, 3-oxy-6-methyl-2-ethylpyridine fumarate, 3-oxy-6-methyl-2-ethylpyridine hydrochloride.

3. Composition according to claim 2, **characterized in that** the derivative of 3-oxypyridine is 3-oxy-6-methyl-2-ethylpyridine hydrochloride or 3-oxy-6-methyl-2-ethylpyridine succinate.

4. Composition according to claim 3, **characterized in that** the derivative of 3-oxypyridine is 3-oxy-6-methyl-2-ethylpyridine succinate.

5. Composition according to claim 1, **characterized in that** it comprises inosine as a purine derivative.

6. Composition according to claim 1, **characterized in that** it comprises the components at following ratios, mg:
3-oxypyridine or a derivative of 3-oxypyridine, approximately 25 mg, to approximately 1,500 mg, nicotinamide-adenine dinucleotide from approximately 0.5 mg to approximately 100 mg of purine or a derivative of it from approximately 100 mg to approximately 1,200 mg.

7. Composition according to claim 6, **characterized in that** it further comprises L-carnitine or its derivative in an amount from approximately 10 mg to approximately 100 mg.

8. Composition according to claim 7, **characterized in that** it comprises L-acetylcarnitine as a derivative of L-carnitine.

9. Composition according to claim 6, **characterized in that** it further comprises choline alphosceratus in an amount from approximately 50 mg to approximately 1,000 mg.

10. Composition from any one of the claims 1 to 9, **characterized in that** it further comprises a pharmaceutically acceptable carrier.

11. Composition according to claim 10, **characterized in that** it is in an injectable medicinal form.

12. Composition according to claim 11, **characterized in that** it is in a form of lyophilizate.

13. Composition according to claim 10, **characterized in that** it is in a solid dosage form.

14. Composition according to claim 13, **characterized in that** it is in a form of a tablet or a coated tablet.

15. Composition according to claim 13, **characterized in that** it is in a form of a solid capsule.

16. Composition according to claim 10, **characterized in that** it is in a form of suppository.

17. Use of a composition comprising
at least one 3-oxypyridine or derivative of 3-oxypyridine, which derivative is chosen from 3-oxy-2-methoxypyridirie, 3-oxy-2-phenylpyridine, 3-oxy-6-methyl-2-ethylpyridine and pharmaceutically acceptable salts thereof,
at least one purine or derivative of purine, chosen from the group consisting of inosine, adenosine, and hypoxanthine, and
nicotinamide-adenine dinucleotide,
for the manufacture of a medicament for treatment of hypoxia, chronic poisoning, ischemic and reperfusion damage, atherosclerosis, arterial hypertension, congestive and chronic cardiac failure.

## Patentansprüche

1. Zusammensetzung, **dadurch gekennzeichnet, dass** sie das Folgende umfasst:
mindestens ein 3-Oxypyridin oder 3-Oxypyridinderivat, wobei das Derivat aus 3-Oxy-2-methoxypyridin, 3-Oxy-2-phenylpyridin, 3-Oxy-6-methyl-2-ethylpyridin und pharmazeutisch unbedenklichen Salzen dieser ausgewählt ist,
mindestens ein Purin oder Purinderivat, welches aus der Gruppe ausgewählt ist, die Inosin, Adenosin und Hypoxanthin umfasst, und
Nicotinamid-Adenin-Dinucleotid,
zur Verwendung bei der Behandlung von Hypoxie, chronischer Vergiftung, Ischämie- und Reperfusionsschäden, Atherosklerose, arteriellem Hochdruck, kongestiver und chronischer Herzinsuffizienz.

2. Zusammensetzung nach Anspruch 1, **dadurch gekennzeichnet, dass** das 3-Oxypyridinderivat aus 3-Oxy-6-methyl-2-ethylpyridinsuccinat, 3-Oxy-6-methyl-2-ethylpyridinfumarat und 3-Oxy-6-methyl-2-ethylpyridinhydrochlorid ausgewählt ist.

3. Zusammensetzung nach Anspruch 2, **dadurch gekennzeichnet, dass** es sich bei dem 3-Oxypyridinderivat um 3-Oxy-6-methyl-2-ethylpyridinhydrochlorid oder 3-Oxy-6-methyl-2-ethylpyridinsuccinat handelt.

4. Zusammensetzung nach Anspruch 3, **dadurch gekennzeichnet, dass** es sich bei dem 3-Oxypyridinderivat um 3-Oxy-6-methyl-2-ethylpyridinsuccinat handelt.

5. Zusammensetzung nach Anspruch 1, **dadurch gekennzeichnet, dass** sie Inosin als Purinderivat umfasst.

6. Zusammensetzung nach Anspruch 1, **dadurch gekennzeichnet, dass** sie die Komponenten in den folgenden Mengenverhältnissen in mg umfasst:
ungefähr 25 mg bis ungefähr 1.500 mg 3-Oxypyridin oder 3-Oxypyridinderivat, ungefähr 0,5 mg bis ungefähr 100 mg Nicotinamid-Adenin-Dinukleotid, ungefähr 100 mg bis ungefähr 1.200 mg Purin oder Purinderivat.

7. Zusammensetzung nach Anspruch 6, **dadurch gekennzeichnet, dass** sie ferner L-Carnitin oder ein Derivat davon in einer Menge von ungefähr 10 mg bis ungefähr 100 mg umfasst.

8. Zusammensetzung nach Anspruch 7, **dadurch gekennzeichnet, dass** sie L-Acetylcarnitin als Derivat des L-Carnitins umfasst.

9. Zusammensetzung nach Anspruch 6, **dadurch gekennzeichnet, dass** sie ferner Cholinalfoscerat oder ein Derivat davon in einer Menge von ungefähr 50 mg bis ungefähr 1.000 mg umfasst.

10. Zusammensetzung nach einem der Ansprüche 1 bis 9, **dadurch gekennzeichnet, dass** sie ferner einen pharmazeutisch unbedenklichen Trägerstoff umfasst.

11. Zusammensetzung nach Anspruch 10, **dadurch gekennzeichnet, dass** sie in einer injizierbaren medizinischen Form vorliegt.

12. Zusammensetzung nach Anspruch 11, **dadurch gekennzeichnet, dass** sie in der Form eines Lyophilisats vorliegt.

13. Zusammensetzung nach Anspruch 10, **dadurch gekennzeichnet, dass** sie in einer festen Dosierungsform vorliegt.

14. Zusammensetzung nach Anspruch 13, **dadurch gekennzeichnet, dass** sie in der Form einer Tablette oder einer beschichteten Tablete vorliegt.

15. Zusammensetzung nach Anspruch 13, **dadurch gekennzeichnet, dass** sie in der Form einer festen Kapsel vorliegt.

16. Zusammensetzung nach Anspruch 10, **dadurch gekennzeichnet, dass** sie in der Form eines Suppositoriums vorliegt.

17. Verwendung einer Zusammensetzung, welche das Folgende umfasst:
mindestens ein 3-Oxypyridin oder 3-Oxypyridinderivat, wobei das Derivat aus 3-Oxy-2-methoxypyridin, 3-Oxy-2-phenylpyridin, 3-Oxy-6-methyl-2-ethylpyridin und pharmazeutisch unbedenklichen Salzen dieser ausgewählt ist,
mindestens ein Purin oder Purinderivat, welches aus der Gruppe ausgewählt ist, die Inosin, Adenosin und Hypoxanthin umfasst, und
Nicotinamid-Adenin-Dinucleotid,
zur Herstellung eines Medikaments zur Behandlung von Hypoxie, chronischer Vergiftung, Ischämie- und Reperfusionsschäden, Atherosklerose, arteriellem Hochdruck, kongestiver und chronischer Herzinsuffizienz.

## Revendications

1. Composition **caractérisée en ce qu'**elle comprend :
au moins une 3-oxypyridine ou un dérivé de 3-oxypyridine, lequel dérivé est choisi parmi la 3-oxy-2-méthoxypyridine, la 3-oxy-2-phénylpyridine, la 3-oxy-6-méthyl-2-éthylpyridine et leurs sels pharmaceutiquement acceptables,
au moins une purine ou un dérivé de purine, choisi dans le groupe constitué par l'inosine, l'adénosine et l'hypoxanthine, et
un dinucléotide nicotinamide-adénine,
destinée à être utilisée dans le traitement de l'hypoxie, d'une intoxication chronique, de lésions ischémiques et de reperfusion, de l'athérosclérose, de l'hypertension artérielle, de l'insuffisance cardiaque congestive et chronique.

2. Composition selon la revendication 1, **caractérisée en ce que** le dérivé de 3-oxypyridine est choisi parmi le succinate de 3-oxy-6-méthyl-2-éthylpyridine, le fumarate de 3-oxy-6-méthyl-2-éthylpyridine, le chlorhydrate de 3-oxy-6-méthyl-2-éthylpyridine.

3. Composition selon la revendication 2, **caractérisée en ce que** le dérivé de 3-oxypyridine est le chlorhydrate de 3-oxy-6-méthyl-2-éthylpyridine ou le succinate de 3-oxy-6-méthyl-2-éthylpyridine.

4. Composition selon la revendication 3, **caractérisée en ce que** le dérivé de 3-oxypyridine est le succinate de 3-oxy-6-méthyl-2-éthylpyridine.

5. Composition selon la revendication 1, **caractérisée en ce qu'**elle comprend de l'inosine en tant que dérivé de la purine.

6. Composition selon la revendication 1, **caractérisée en ce qu'**elle comprend les composants aux rapports suivants, mg :
3-oxypyridine ou un dérivé de 3-oxypyridine, d'approximativement 25 mg à approximativement 1 500 mg, dinucléotide nicotinamide-adénine d'approximativement 0,5 mg à approximativement 100 mg, purine ou un dérivé de celle-ci d'approximativement 100 mg à approximativement 1 200 mg.

7. Composition selon la revendication 6, **caractérisée en ce qu'**elle comprend en outre de la L-carnitine ou son dérivé en une quantité d'approximativement 10 mg à approximativement 100 mg.

8. Composition selon la revendication 7, **caractérisée en ce qu'**elle comprend de la L-acétylcarnitine en tant que dérivé de la L-carnitine.

9. Composition selon la revendication 6, **caractérisée en ce qu'**elle comprend en outre de l'alphoscératus de choline en une quantité d'approximativement 50 mg à approximativement 1 000 mg.

10. Composition selon l'une quelconque des revendications 1 à 9, **caractérisée en ce qu'**elle comprend en outre un vecteur pharmaceutiquement acceptable.

11. Composition selon la revendication 10, **caractérisée en ce qu'**elle se trouve sous une forme médicale injectable.

12. Composition selon la revendication 11, **caractérisée en ce qu'**elle se trouve sous la forme d'un lyophilisat.

13. Composition selon la revendication 10, **caractérisée en ce qu'**elle se trouve sous une forme posologique solide.

14. Composition selon la revendication 13, **caractérisée en ce qu'**elle se trouve sous la forme d'un comprimé ou d'un comprimé pelliculé.

15. Composition selon la revendication 13, **caractérisée en ce qu'**elle se trouve sous la forme d'une gélule solide.

16. Composition selon la revendication 10, **caractérisée en ce qu'**elle se trouve sous la forme d'un suppositoire.

17. Utilisation d'une composition qui comprend:
au moins une 3-oxypyridine ou un dérivé de 3-oxypyridine, lequel dérivé est choisi parmi la 3-oxy-2-méthoxypyridine, la 3-oxy-2-phénylpyridine, la 3-oxy-6-méthyl-2-éthylpyridine et leurs sels pharmaceutiquement acceptables,
au moins une purine ou un dérivé de purine, choisi dans le groupe constitué par l'inosine, l'adénosine et l'hypoxanthine, et
un dinucléotide nicotinamide-adénine,
pour la fabrication d'un médicament destiné au traitement de l'hypoxie, d'une intoxication chronique, de lésions ischémiques et de reperfusion, de l'athérosclérose, de l'hypertension artérielle, de l'insuffisance cardiaque congestive et chronique.
